(19) Europäisches Patentamt

European Patent Office

Office européen des brevets

(11) Veröffentlichungsnummer: **0 278 284 B1**

(12) **EUROPÄISCHE PATENTSCHRIFT**

(45) Veröffentlichungstag der Patentschrift: **22.05.91**

(51) Int. Cl.⁵: **C09B** 61/00, A23L 1/275

(21) Anmeldenummer: **88100886.6**

(22) Anmeldetag: **22.01.88**

(54) **Pulverförmige, wasserdispergierbare Carotinoid-Zubereitungen und Verfahren zu ihrer Herstellung.**

(30) Priorität: **24.01.87 DE 3702030**

(43) Veröffentlichungstag der Anmeldung:
**17.08.88 Patentblatt 88/33**

(45) Bekanntmachung des Hinweises auf die
Patenterteilung:
**22.05.91 Patentblatt 91/21**

(84) Benannte Vertragsstaaten:
**AT BE CH DE ES FR GB GR IT LI NL SE**

(56) Entgegenhaltungen:
**EP-A- 0 065 193          DE-B- 1 211 911**
**FR-A- 2 281 961          FR-A- 2 578 719**
**US-A- 2 861 891          US-A- 4 316 917**

**CHEMICAL ABSTRACTS, Band 53, 1959, Zusammenfassung Nr. 2498c, Columbus, Ohio, US; R.H. BUNNELL et al.: "Coloring waterbase foods with beta-carotene", & FOOD TECHNOL. 12, 536-41 (1958)**

(73) Patentinhaber: **BASF Aktiengesellschaft**
**Carl-Bosch-Strasse 38**
**W-6700 Ludwigshafen(DE)**

(72) Erfinder: **Horn, Dieter, Dr.**
**Schroederstrasse 69**
**W-6900 Heidelberg(DE)**
Erfinder: **Lueddecke, Erik, Dr.**
**Thomas-Mann-Strasse 27**
**W-6704 Mutterstadt(DE)**
Erfinder: **Schaefer, Peter, Dr.**
**Im Buegen 16**
**W-6719 Kirchheim(DE)**

## Beschreibung

Die Erfindung betrifft die Überführung von Carotinoiden in eine feinverteilte, pulverförmige Form, wobei das Carotinoid in einem eßbaren Öl gelöst ist und die Öllösung in Form kleinster Öltropfen vorliegt. Diese Zubereitung dient insbesondere zum Färben von Lebens-und Futtermitteln.

Die Carotinoide bilden eine Gruppe von Farbpigmenten mit gelber bis roter Farbtonnuance, die in der Natur weitverbreitet vorkommen und vielen Nahrungsmitteln eine charakteristische Färbung verleihen. Als wichtigste Vertreter dieser Stoffklasse seien β-Carotin, β-Apo-8′-carotinal, Canthaxanthin und Citranaxanthin genannt. Sowohl für die Lebensmittel-und Futtermittelindustrie als auch für die pharmazeutische Technologie stellen diese synthetisch herstellbaren Substanzen z. B. als Ersatz für künstliche Farbstoffe wichtige Farbkörper dar und sind z. B. wegen ihrer Pro-Vitamin-A-Aktivität von Interesse.

Nahezu alle Carotinoide sind in Wasser unlöslich, während in Fetten und Ölen eine ebenfalls nur geringe Löslichkeit gefunden wird. Diese begrenzte Löslichkeit sowie die hohe Oxidationsempfindlichkeit behindern eine direkte Anwendung der relativ grobkörnigen bei der Synthese erhaltenen Produkte in der Einfärbung von Lebens- oder Futtermitteln, da nur eine geringe Farbausbeute erzielt werden kann und die Substanzen in grobkristalliner Form nur schlecht resorbiert werden. Diese für die praktische Versendung der Carotinoide nachteiligen Effekte wirken sich insbesondere im wäßrigen Medium aus, da die meisten darin gänzlich unlöslich sind.

Zur Verbesserung der Farbausbeuten und zur Erhöhung der Resorbierbarkeit sind verschiedene Verfahren beschrieben worden, die alle das Ziel haben, die Kristallitgröße der Wirkstoffe zu verkleinern und auf einen Teilchengrößenbereich von kleiner 10μm zu bringen. So kann z. B. nach Chimia 21, 329 (1967), β-Carotin zusammen mit Speiseöl unter Stickstoffatmosphäre in einer Kolloidmühle bis aus eine Partikelgröße von 2 bis 5μm vermahlen werden. Gemäß Food. Technol. 12, 527 (1958), bewirkt dabei das umhüllende Öl gleichzeitig einen Oxidationsschutz für den Wirkstoff. Eine so erhaltene Suspension, die bis zu 20 oder 30 % Wirkstoff enthält, kann mit Erfolg zur Einfärbung von Fetten und Ölen benutzt werden, da trotz der geringen Löslichkeit diese ausreicht, um die Kristalle bei der üblicherweise angewandten geringen Konzentration in Lösung zu bringen.

Während die Anfärbung von öligen bzw. fettigen Systemen mit den reinen kristallinen Substanzen somit gut möglich ist, können jedoch wäßrige Systeme damit praktisch nicht gefärbt werden. Auch die Verwertung von der Nahrung bzw. dem Futter biegemischten reinen Carotinoiden durch den menschlichen bzw. tierischen Organismus ist wegen der völligen Unlöslichkeit der meisten Carotinoide in Wasser sehr schlecht. Selbst in organischen Lösungsmitteln wie Alkoholen und Alkanen ist die Löslichkeit der Carotinoide nur sehr begrenzt.

Die gewünschten Färbe- und Resorptionseigenschaften können nur über den möglichst feinverteilten Zustand erzielt werden. Wünschenswert ist dabei eine Partikelgröße von kleiner als 1μm, die durch Vermahlung entweder überhaupt nicht oder nur unter Schädigung des Wirkstoffs erreichbar ist.

Einen gewissen Fortschritt demgegenüber stellen vorbekannte Verfahren dar, bei denen man den Wirkstoff in einem mit Wasser nicht mischbaren Lösungsmittel, vorzugsweise einem Chlorkohlenwasserstoff wie Chloroform oder Methylenchlorid löst, die Lösung durch Homogenisieren in einer Gelatine-Zucker-Lösung emulgiert und aus der Emulsion schließlich das Lösungsmittel abzieht, wobei der Wirkstoff in feinkristalliner Form freigesetzt wird. Dieses Verfahren ist in Chimia 21, 329 (1967), sowie in DE-AS 12 11 911 und DE-OS 25 34 091 beschrieben. Aus der erhaltenen Suspension wird dann durch Entwässern ein feinverteiltes Pulver gewonnen.

Das vorgenannte Verfahren hat aber den Nachteil, daß chlorierte Kohlenwasserstoffe verwendet werden müssen, um eine ausreichend hohe Wirkstoffkonzentration in der Emulsionsphase zu erzielen. Die vollständige Entfernung der chlorierten Kohlenwasserstoffe, die aus toxikologischen Gründen erforderlich ist, kann aber technisch nur schwer erzielt werden.

Diese Nachteile wurden gemäß dem Verfahren der europäischen Patentschrift 0065 193 überwunden, bei dem man ein Carotinoid in einem flüchtigen, mit Wasser mischbaren organischen Lösungsmittel bei Temperaturen zwischen 50°C und 200°C, gegebenenfalls unter erhöhtem Druck, innerhalb einer Zeit von weniger als 10 Sekunden löst, aus der erhaltenen molekulardispersen Lösung sofort durch schnelles Mischen mit einer wäßrigen Lösung eines quellbaren Kolloids bei Temperaturen zwischen 0°C und 50°C das Carotinoid in kolloiddisperser Form ausfällt und die erhaltene Dispersion in an sich bekannter Weise von dem Lösungsmittel und dem Dispergiermedium befreit. Die mittlere Größe der auf diesem Wege erzeugten Carotinoid-Feststoffteilchen liegt bei Werten unterhalb 0,3μm.

Nach einem anderen Verfahren (vgl. US-Patentschrift 1 861 891 und Öster. Patentschrift 202 273) wird eine wasserdispergierbare Carotinoidzubereitung dadurch hergestellt, daß man bei 100 bis 160°C eine übersättigte Lösung des Carotinoides in einem eßbaren und bei etwa 20 bis 40°C flüssigen Öl herstellt, diese übersättigte Lösung in einem wässrigen, gelatinösen Material emulgiert und die Emulsion in bekannter Weise in trockene Kleinteilchen überführt.

Wird das Trockenpulver in warmem Wasser redispergiert, so bildet sich erneut eine trübe, orangegelbe Emulsion. Diese kann z. B. zum Färben von Lebensmitteln eingesetzt werden. Wichtige anwendungstechnische Eigenschaften dieser Färbepräparate sind Löslichkeit, Farbton, Farbstärke, Trübung und Stabilität im Anwendungsmedium. (R.H. Bunnell, W. Driscoll, I.C. Bauernfeind, Food, Technol. XII, 1 - 81, 1958).

Eine spektralphotometrische Untersuchung der nach dem aufgeführten Stand der Technik erzeugten feinverteilten Carotinoid-Zubereitungen zeigt jedoch, daß besonders bei höheren Konzentrationen, z.B. ≧ 2% im Trockenpulver, die Extinktionswerte am Bandenmaximum der koloristisch wirksamen Absorptionsbanden im sichtbaren Spektralbereich bei Werten liegen, die nur 50 % der maximal in einer echten Lösung erzielbaren Werte darstellen. Aus wirtschaftlicher Sicht stellt dies einen erheblichen Nachteil dar, da die potentiell verfügbare Farbstärke eines Carotinoids z. B. bei der Einfärbung eines Lebensmittels nur zu 50 % genutzt werden kann und somit zur Erzielung eines angestrebten Farbstärkewertes die Dosierung des Farbstoffs verdoppelt werden muß. Weiterhin ist bekannt, daß der Farbton eines mit Carotinoiden eingefärbten Lebensmittels stark durch die Teilchengröße und den physikalischen Aggregatzustand (Festkörper, Lösung) bestimmt wird. Mit den Produkten des Standes der Technik wird die Breite der potentiell möglichen Unterschiede im Farbton eines ausgewählten Carotinoids bei weitem nicht ausgeschöpft. Es ist weiterhin bekannt, daß die biologische Resorption wasserunlöslicher Wirkstoffe, z.B. nach oraler Applikation, stark von der Teilchengröße und dem phys. Aggregationszustand beeinflußt wird. Die nach dem Stand der Technik erzeugten Carotinoid-Präparate erfüllen deshalb nicht die Voraussetzung für eine optimale biologische Resorption.

Es bestand daher die Aufgabe, ein Verfahren vorzuschlagen, nach dem die Herstellung von Carotinoid-Formulierungen gelingt, die nicht die genannten Nachteile aufweisen.

Diese Aufgabe wurde erfindungsgemäß dadurch gelöst, daß man das Carotinoid in einem flüchtigen, mit Wasser mischbaren, organischen Lösungsmittel bei Temperaturen zwisxhen 50 und 240°C, vorzugsweise zwischen 150 und 200°C, gemeinsam mit der 1,5- bis 20fachen Gewichtsmenge, bezogen auf das Carotinoid eines eßbaren Öls, sowie einem Emulgator, gegebenenfalls unter erhöhtem Druck, rasch löst, aus der erhaltenen molekulardispersen Lösung durch schnelles Mischen mit einer wäßrigen Lösung eines Schutzkolloids bei Temperaturen zwischen 0°C und 50°C die hydrophile Lösungsmittelkomponente in die wäßrige Phase überführt, wobei die hydrophobe Ölphase, die das Carotinoid gelöst enthält, als mikrodisperse Phase entsteht, und die erhaltene Zweiphasen-Mischung in an sich bekannter Weise von dem Lösungsmittel und dem Wasser befreit, so daß man ein trockenes Pulver mit einem Gehalt von 5 bis 50 Gew.% eines eßbaren Öls erhält.

Die erfindungsgemäße Arbeitsweise macht sich dem Umstand zunutze, daß die in der Kälte sehr geringe Löslichkeit der Carotinoide in einer Lösung eines eßbaren Öls in wassermischbaren Lösungsmitteln mit steigender Temperatur deutlich zunimmt, jedoch trotz der hohen Temperaturen infolge der nur kurzen Verweilzeit bei hoher Temperatur eine den Farbton, die Farbstärke sowie die biologische Wirksamkeit beeinflussende Isomerisierung weitgehend unterbleibt. Dies stand allerdings im Widerspruch zu dem Umstand, daß gerade die beim Erhitzen in heißem Öl üblicherweise eintretende Isomerisierung dazu benutzt wird, eine den Farbton sowie die Farbstärke des angestrebten Endproduktes beeinträchtigende Rekristallisation in den Tröpfchen der emulgierten Ölphase beim Absinken der Temperatur zu inhibieren. Es war daher überraschend, daß beim Vorgehen nach der erfindungsgemäßen Verfahrensweise trotz Unterdrückung der Isomerisierungstendenz bei hohen Temperaturen nach Abkühlung ein Produkt erhalten wird, das das Carotinoid spektralphotometrisch nachweisbar als molekulardisperse, gegen Rekristallisation stabilisierte, übersättigte Öllösung in Form submikroskopisch kleiner Ölteilchen enthält, und das gegenüber Produkten, hergestellt nach dem Stand der Technik, eine bis zu 100%ige Steigerung der Farbstärke bei gleichzeitiger Verschiebung der Farbtonnuance in bisher unzugängliche Bereiche aufweist. Die photochemische Stabilität der nach dem erfindungsgemäßen Verfahren hergestellten Hydrosole übertrifft die der nach dem Stand der Technik hergestellten Produkte um mehr als das 5-fache.

Die Carotinoide, die bei der Durchführung der Erfindung eingesetzt werden können, sind die bekannten, zugänglichen, natürlichen oder synthetischen Vertreter dieser Klasse von Verbindungen, die als farbgebende Mittel brauchbar sind, z.B. Carotin, Lycopin, Bixin, Zeaxanthin, Cryptoxanthin, Citranaxanthin, Lutein, Canthaxanthin, Astaxanthin, β-Apo-4'-carotinal, β-Apo-8'-carotinal, β-apo-12'-carotinal, β-Apo-8'-carotinsäure sowie Ester von hydroxy- und carboxyhaltigen Vertretern dieser Gruppe, z.B. die niederen Alkylester und vorzugsweise die Methyl- und Ethylester. Besonders bevorzugt werden die bisher technisch gut zugänglichen Vertreter wie β-Carotin, Canthaxanthin, β-Apo-8'-carotinal und β-Apo-8'Carotinsäureester.

Zur Durchführung des erfindungsgemäßen Verfahrens sind vor allem wassermischbare, thermisch stabile, flüchtige, nur Kohlenstoff, Wasserstoff und Sauerstoff enthaltende Lösungsmittel wie Alkohole, Ether, Ester, Ketone und Acetale geeignet. Vorzugsweise werden Ethanol, n-Propanol, Isopropanol, 1,2-Butandiol-1-methylether, 1,2-Propandiol-1-n-propylether oder Aceton verwendet.

Allgemein verwendet man zweckmäßig solche Lösungsmittel, die mindestens zu 10 % wassermischbar sind, einen Siedepunkt unter 100°C aufweisen und/oder weniger als 10 Kohlenstoffatome haben.

Als eßbare Öle kommen Öle in Betracht, die bei 20 bis 40°C flüssig sind. Beispielsweise sind pflanzliche Öle, wie Maisöl, Kokosöl, Sesamöl, Arachisöl, Sojabohnenöl, Erdnußöl oder Baumwollsamenöl zu nennen. Besonders bevorzugt ist Erdnußöl. Andere geeignete Öle oder Fette sind Schweineschmalz, Rindertalg und Butterfett. Die eßbaren Öle werden im allgemeinen in der 1,5- bis 20-, vorzugsweise 3- bis 8-fachen Gewichtsmenge, bezogen auf das Carotinoid angewandt, wobei der Gesamtölgehalt der Carotinoid-Zubereitung 60 Gew.-% nicht überschreiten sollte, wenn ein Trockenpulver hergestellt werden soll.

Als Schutzkolloide kommen beliebige, übliche in Nahrungs- und Futtermitteln zugelassene Schutzkolloide in Betracht; beispielsweise werden Gelatine, Stärke, Dextrin, Dextran, Pektin, Gummiarabicum, Kasein, Kaseinat, Vollmilch, Magermilch, Milchpulver oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fast Soluble Vitamins, Intern. Encyclopedia of Food and Nutrition, Bd. 9, Pergamon Press 1970, S. 128 - 131, verwiesen. Zur Erhöhung der mechanischen Stabilität des Endproduktes ist es zweckmäßig, dem Kolloid einen Weichmacher zuzusetzen, wie Zucker oder Zuckeralkohole, z.B. Saccharose, Glukose, Lactose, Invertzucker, Sorbit, Mannit oder Glycerin. Als Konservierungsstoffe können noch untergeordnete Mengen an Methylester oder Propylester der p-Hydroxybenzoesäure, Sorbinsäure und Na-Benzoat zugefügt werden.

Das Verhältnis Schutzkolloid, Weichmacher und Öl zu Carotinoid wird im allgemeinen so gewählt, daß ein Endprodukt erhalten wird, das zwischen 0,5 und 10 Gew.%, vorzugsweise 2 bis 5 %, Carotinoid, 5 bis 50 % eines eßbaren Öls, 10 bis 50 Gew.% eines Schutzkolloids, 20 bis 70 % eines Weichmachers, alle Prozentangaben bezogen auf die Trockenmasse des Pulvers, sowie untergeordnete Mengen eines Stabilisators enthält, wobei die mittlere Teilchengröße der im Pulver vorliegende mit molekulardispersem Carotinoid übersättigten Ölphase bei weniger als 0,3 μm und einer Halbwertsbreite der Größenverteilung von weniger als 50 % liegt. Das Produkt enthält praktisch keine Ölteilchen mit einer Korngröße über 1 μm.

Zur Erhögung der Stabilität des Wirkstoffes gegen oxidativen Abbau ist es vorteilhaft, Stabilisatoren wie α-Tocopherol, Lecithin, 5-Butyl-hydroxytoluol, t-Butylhydroxyanisol, Ethoxyquine oder Ascorbylpalmitat zuzusetzen.

Sie können entweder der wäßrigen oder der Lösungsmittel-Phase zugesetzt werden, vorzugsweise werden sie jedoch gemeinsam mit den Farbstoffen und dem Öl in der Lösungsmittel-Phase gelöst.

Nach dem erfindungsgemäßen Verfahren erhält man eine tiefgefärbte viskose Flüssigkeit, aus der die Entfernung des Lösungsmittels je nach Siedepunkt in an sich bekannter Weise z. B. durch Destillation, gegebenenfalls unter vermindertem Druck, oder durch Extraktion mit einem mit Wasser nicht mischbaren Lösungsmittel erfolgen kann. Vorzugsweise erfolgt sie jedoch gemeinsam mit der Entfernung des Wassers durch Sprühtrocknung und Sprühgranulation.

Man erhält ein Trockenpulver, das erneut in Wasser unter Erzielung einer gleichmäßigen Feinverteilung des Wirkstoffs im Korngrößenbereich <0,5 μm gelöst werden kann. Im photochemischen Stabilitätstest erweist sich das so erhaltene Wirkstoff-Hydrosol trotz der Feinverteilung als außerordentlich stabil.

Gegebenenfalls kann die mikrodisperse, mit Carotinoid übersättigte Ölphase auch nach Einstellung eines geeigneten pH-Wertes gemeinsam mit dem Schutzkolloid ausgeflockt und damit in eine Form überführt werden, aus der durch Filtrieren oder Zentrifugieren auf einfache Weise das Lösungsmittel und ein Großteil des Dispergiermediums abgetrennt werden kann. Das so gewonnene Koazervat wird dann in an sich bekannter Weise nachgetrocknet und in ein Granulat überführt.

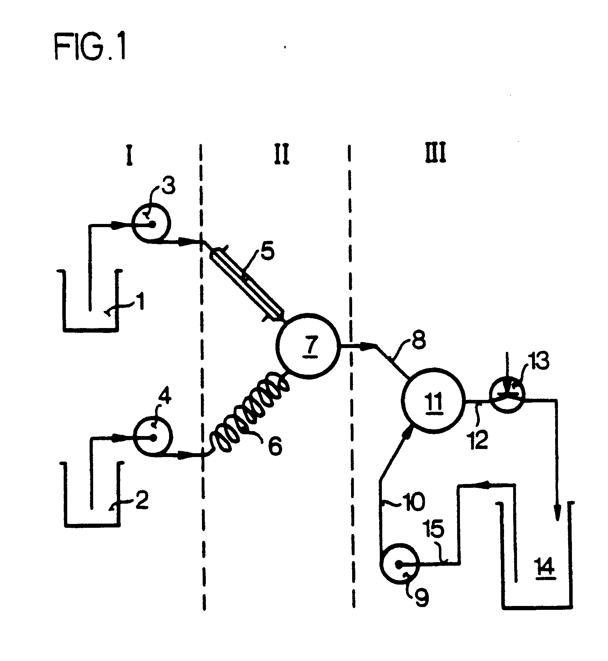

Im einzelnen führt man das erfindungsgemäße Verfahren beispielsweise mit einer Apparatur, wie sie in Fig. 1 schematisch dargestellt ist, wie folgt durch:
Die Apparatur gliedert sich in die Teile I, II und III. Teil II is der Hochtemperaturabschnitt, während in den Teilen I und III die Temperaturen weniger als 50°C betragen.

Im Gefäß (1) wird eine Suspension des Carotinoids gemeinsam mit dem Öl in dem gewählten Lösungsmittel in einer Konzentration von 2 bis 20 Gew.%, bezogen auf die Mischung, gegebenenfalls unter Zusatz von 0,1 bis 10 Gew.% an Stabilisatoren, vorgelegt. Gefäß (2) enthält das Lösungsmittel ohne Beimischung des Carotinoids. Über die Pumpen (3) bzw. (4) werden die Wirkstoff-Suspensionen und das Lösungsmittel der Mischkammer (7) zugeführt, wobei das Mischungsverhältnis durch Wahl der jeweiligen Förderleistung der Pumpen vorgegeben werden kann und so gewählt wird, daß je nach Lösungsmittel und Verweilzeit eine Carotinoid-Konzentration in der Mischkammer von 0,5 bis 10 Gew.%, bezogen auf die Lösung, entsteht. Das Mischkammervolumen (7) ist so bemessen, daß bei der gewählten Förderleistung der Pumpen (3) und (4) die Verweilzeit in (7) vorzugsweise weniger als 1 Sekunde beträgt.

Das Lösungsmittel wird vor Eintritt in die Mischkammer über den Wärmetauscher (6) auf die gewünschte Temperatur gebracht, während die ölhaltige Wirkstoffsuspension durch Zuführung über die thermisch isolierte Zuleitung (5) bei Temperaturen unterhalb 50° C gehalten wird. Durch turbulente Mischung in (7) erfolgt im Temperaturbereich 50 bis 240° C, vorzugsweise jedoch bei 150 bis 200° C, die Lösung des Wirkstoffes, und die erhaltene Lösung tritt über (8) nach kurzer Verweilzeit, vorzugsweise von weniger als einer Sekunde, in die zweite Mischkammer (11) ein, in der durch Zumischen einer wässrigen Schutzkolloid-Weichmacher-Lösung, über die Pumpe (9) und die Zuleitung (10) die Zerlegung der molekulardispersen Carotinoidlösung in ein Zweiphasengemisch unter Bildung einer mikrodispersen den Wirkstoff in übersättigter Lösung enthaltenden Ölphase und einer homogenen, das wassermischbare Lösungsmittel enthaltenden wäßrigen Phase, erfolgt. Über Leitung (12) wird sodann das mikrodisperse Zweiphasengemisch über das Überdruckventil ausgetragen und dem Vorratsgefäß (14) zugeführt. Zur Erzielung einer möglichst hohen Wirkstoffkonzentration kann die Dispersion über die Saugleitung (15) im Kreis geführt werden.

Bei Einstellung des Überdruckventils (13) auf Drücke oberhalb ein bar können in dem neuen Verfahren sogar Lösungsmittel bei Temperaturen oberhalb ihres Siedepunktes (bei Normaldruck) verwendet werden.

Aus der Dispersion kann ein pulverförmiges Präparat in an sich bekannter Weise, z. B. gemäß den Angaben der DE-OS 25 34 091 durch Sprühtrocknen oder durch Sprühkühlen oder durch Einhüllen der Teilchen, Abtrennen und Trocknen im Wirbelbett erfolgen.

Zum Sprühtrocknen wird die Dispersion entweder zuerst vom Lösungsmittel durch Destillation, vorzugsweise unter vermindertem Druck, oder durch Extraktion mit einem mit Wasser nicht mischbaren Lösungsmittel befreit oder die gesamte Mischung wird sprühgetrocknet und so Wasser und Lösungsmittel zusammen im Srpühturm abgezogen.

Am Boden des Sprühturms fällt das Carotinoidpulver entweder bereits trocken oder rieselfähig an. In manchen Fällen kann es zweckmäßig sein, die vollständige Trocknung zusätzlich in einem Wirbelbett vorzunehmen. Anstelle der Herstellung der Pulverzubereitung durch Sprühtrocknen können auch beliebige andere Methoden angewendet werden, um die in der Wasser/Öl/Lösungsmittel-Dispersion bereits feinverteilten Carotinoide in die Pulverform zu überführen.

Ein bekanntes und hier gleichermaßen anwendbares Verfahren besteht z. B. darin, die vom Lösungsmittel befreite Dispersion mit Paraffinöl zu emulgieren, die Mischung abzukühlen, das Paraffinöl von den eingehüllten Carotinoidteilchen zu trennen, das erhaltene Carotinoidpräparat mit Benzin zu waschen und im Wirbelbett zu trocknen.

Bei der erfindungsgemäßen Arbeitsweise war es besonders überraschend, daß bei Verwendung der genannten wassermischbaren Lösungsmittel in Abmischungen mit einem eßbaren Öl, die gegebenenfalls noch Emulgatoren wie Ascorbylpalmitat, Mono- und Diglyceride, Ester von Monogylceriden mit Essigsäure, Zitronensäure, Milchsäure oder Diacetylweinsäure, Polyglycerinfettsäureester, Sorbitanfettsäureester, Propylenglykol-Fettsäureester, Stearoyl-2-Lactylate oder Lecithin enthalten können, stark übersättigte Lösungen hergestellt werden können, aus denen der Wirkstoff, trotz Unterdrückung der trans-cis-Isomerisierung, in der mikrodispersen Ölphase nach der durch die turbulente Zumischung der wässrigen Schutzkolloidlösung ausgelösten Phasentrennung auch während der Entfernung des flüchtigen Lösungsmittels, z.B. durch Destillation oder Sprühtrocknung, und nach Abkühlung keine Rekristallisation des Carotinoids innerhalb der mit Wirkstoff übersättigten submikroskopisch kleinen Öltröpfchen zeigt.

Es ist weiterhin überraschend, daß durch die Abmischung der lösungsmittelhaltigen Öllösung der Carotinoide mit der wässrigen Schutzkolloidlösung eine Phasentrennung ausgelöst wird, bei der die disperse Ölphase in Form außergewöhnlich kleiner Teilchen anfällt, wie sie durch mechanische Homogenisierung nicht erzielt werden kann. Dieser Feinverteilungszustand der mit Wirkstoff übersättigten Ölphase bleibt auch während der Entfernung des flüchtigen Lösungsmittels, z.B. durch Sprühtrocknen, erhalten. Es ist ohne Schwierigkeiten möglich, Präparate zu erhalten, in denen der Hauptanteil der Ölphase in einer Tröpfchengrösse von 0,2 $\mu$m vorliegt, ohne daß gleichzeitig Tröpfchen von über 1 $\mu$m vorhanden sind. Das Absorptionsspektrum solcher Carotinoid-Präparate zeigt auch nach Sprühtrocknung und Wiederauflösung in einem wäßrigen Medium die für die molekulardisperse Lösung eines Carotinoids in einem eßbaren Öl typische Bandenform und Extinktion.

In den nachfolgenden Beispielen wird die Durchführung des erfindungsgemäßen Verfahrens näher erläutert. Die Angaben zum Stand der Technik beziehen sich auf Präparate erhalten gemäss R.H.BUNNEL et al. Food Technology 12, 536 (1958).

Beispiel 1

5 g β-trans-Carotin werden in 240 g einer Lösung von 4 g Ascorbylpalmitat, 5 g α-Tocopherol gemeinsam mit 20 g Erdnußöl in Isopropanol suspendiert und bei Einstellung des Druckbegrenzungsventils (13) auf 25 bar in der Mischkammer (7) mit 360 g Isopropanol gemischt, das im Wärmetauscher (6) auf 225°C erhitzt wurde. Bei einer Dosierleistung von 2 1/h auf der Suspensionsseite und 3 1/h auf der Lösungsmittelseite beträgt die Verweilzeit in der Mischkammer (7) 0,35 Sekunden. Die dabei bei einer Temperatur von 190°C entstehende molekulardisperse Lösung wird sodann Mischkammer (11) zugeführt, in der durch turbulentes Vermischen mit 4000 g einer mit 1 n NaOH auf pH 9 eingestellten wässrigen Lösung von 60 g Gelatine und 90 g Saccharose bei einer Dosiergeschwindigkeit von 27 1/h eine Phasentrennung unter Bildung einer mikrodispersen Ölphase, die das β-Carotin übersättigt gelöst enthält, stattfindet. Es wird im Auffanggefäß (14) eine mikrodisperse Zweiphasenmischung mit gelber Farbton-Nuance und einer Temperatur von 50°C erhalten. Die Teilchengrößenanalyse durch Protonen-Korrelations-Spektroskopie liefert für die mittlere Teilchengröße der Ölphase einen Wert von 210 nm bei einer Verteilungsbreite von ±40 %.

Nach Abtrennen des Lösungsmittels unter vermindertem Druck bei 50°C in einer Destillationsapparatur wird eine viskose Flüssigkeit erhalten, die durch Sprühtrocknung in ein stabiles, wasserlösliches Trockenpulver überführt werden kann. Der β-Carotin-Gehalt dieses Trockenpulvers beträgt 2,4 Gew.%.

Nach Wiederauflösung des Trockenpulvers in kaltem Wasser wird eine gelbe Lösung erhalten, in der die Ölphase erneut als mikrodisperse Phase bei einer Teilchengröße von 220 nm ±30 % vorliegt.

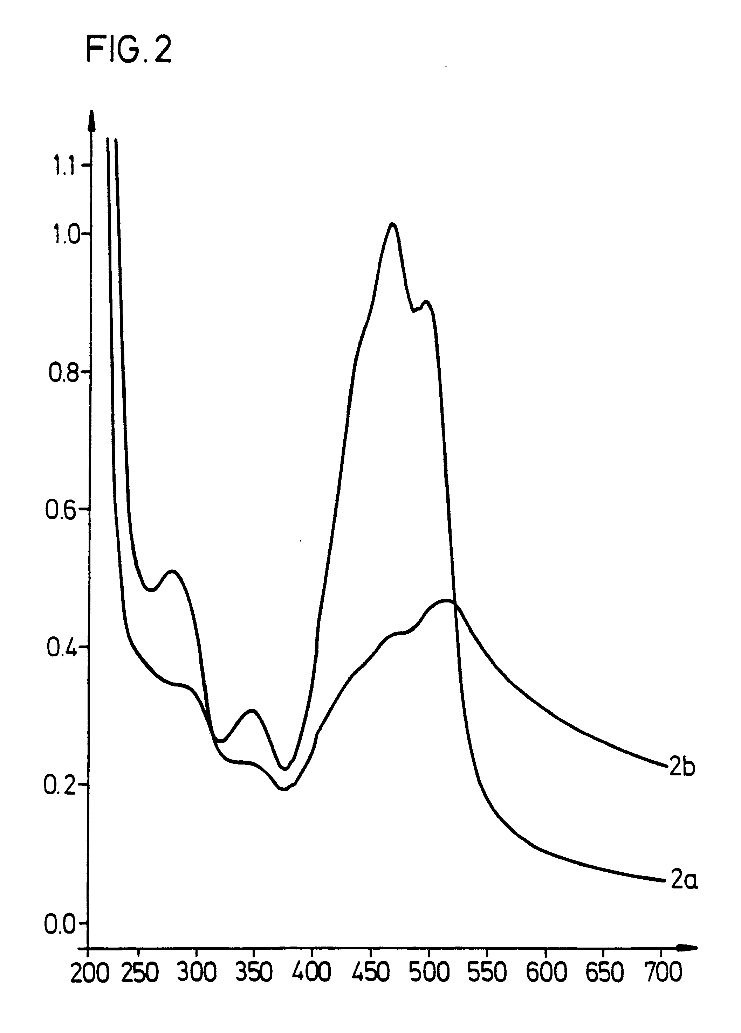

Die spektralphotometrische Untersuchung dieser Lösung zeigt die für eine molekulardisperse Lösung typische Bandenform für β-Carotin (Fig. 2a). Nach dem Stand der Technik wird dagegen ein Produkt erhalten, das bei gleicher Wirkstoffkonzentration in wässriger Lösung ein Absorptionsspektrum liefert, das den typischen Verlauf eines β-Carotin-Festkörperspektrums zeigt (Fig. 2b). Das Verhältnis der Extinktionswerte an den Bandenmaxima liegt bei 2.1. Mit dem erfindungsgmäßen Verfahren wurde somit ein Präparat erhalten, das bezüglich der Farbstärke ein Präparat nach dem Stand der Technik um mehr als das zweifache übertrifft.

Trotz der extremen Feinteiligkeit und der hohen Farbstärke zeigt das Hydrosol im Photostabilitätstest eine hervorragende Stabilität. Unter standardisierten Bestrahlungsbedingungen wird bei einer Bestrahlungszeit von 270 Minuten ein Wirkstoffverlust von 10 % registriert. Bei Produkten mit einem β-Carotin-Gehalt von 2,4 % die nach dem Stand der Technik hergestellt wurden, wird ein Wirkstoffverlust von 10 % unter gleichen Bestrahlungsbedingungen bereits nach 50 Minuten beobachtet.

Beispiel 2

Gemäß den Angaben des Beispiels 1, jedoch unter Einsatz von 10 g β-trans-Carotin, 8 g Ascorbylpalmitat und 40 g Erdnußöl wird eine Zweiphasenmischung erhalten, in der die mit β-Carotin übersättigte Ölphase in Form submikroskopisch kleiner Tröpfchen vorliegt mit einer mittleren Teilchengröße von 249 nm ±52 %.

Das durch Sprühtrocknung erhaltene Trockenpulver hat einen Wirkstoffgehalt von 5 % und liefert nach Wiederauflösen in Wasser ein Hydrosol mit einer mittleren Teilchengröße von 289 nm ±54 %. Im Vergleich mit einem Produkt nach dem Stand der Technik liegt das Extinktionsverhältnis am Bandenmaximum bei 1.8.

Beispiel 3

Nach den Angaben des Beispiels 1, jedoch unter Verwendung von 5 g Canthaxanthin wird ein Trockenpulver erhalten, das nach Wiederauflösen in Wasser ein Hydrosol liefert mit einer mittleren Teilchengröße von 191 nm ±42 %. Der Extinktionswert am Bandenmaximum bei λ = 478 nm liegt bei 90 % des theoretischen Maximalwertes, während Produkte nach dem Stand der Technik lediglich Extinktionswerte liefern, die bei max. 50 % des theoretischen Wertes liegen.

Beispiel 4

Gemäß den Angaben des Beispiels 1, jedoch unter Verwendung von 60 g Gummiarabicum als Schutzkolloid wird ein Trockenpulver erhalten, das nach Auflösen in Wasser ein Hydrosol liefert mit einer mittleren Teilchengröße von 359 nm ±42 %.

## Ansprüche

1. Pulverförmige, wasserdispergierbare Carotinoid-Zubereitungen, die 5 bis 50 Gew.% eines eßbaren Öls enthalten und in denen das Carotinoid in dem eßbaren Öl gelöst und die Öllösung in Form kleiner Tröpfchen die in einer pulverförmigen Matrix dispergiert sind, vorliegen, erhältlich durch rasche Lösung eines Carotinoids in einem flüchtigen, mit Wasser mischbaren, organischen Lösungsmittel bei Temperaturen zwischen 50 und 240° C, gemeinsam mit der 1,5- bis 20-fachen Gewichtsmenge, bezogen auf das Carotinoid, eines eßbaren Öls, sowie einem Emulgator, gegebenenfalls unter erhöhtem Druck, anschließendes sofortiges Mischen mit einer wäßrigen Lösung eines Schutzkolloids bei Temperaturen zwischen 0° C und 50° C und dadurch Überführen der hydrophilen Lösungsmittelkomponente in die wäßrige Phase, wobei die hydrophobe Ölphase, die das Carotinoid gelöst enthält, als mikrodisperse Phase entsteht, und Befreien der erhaltenen Zweiphasen-Mischung in an sich bekannter Weise von dem Lösungsmittel und dem Wasser.

2. Verfahren zur Herstellung von pulverförmigen wasserdispergierbaren Carotinoid-Zubereitungen, die 5 bis 50 Gew.% eines eßbaren Öls enthalten und in denen das Carotinoid in dem eßbaren Öl gelöst und die Öllösung in Form kleiner Tröpfchen vorliegt, dadurch gekennzeichnet, daß man ein Carotinoid in einem flüchtigen, mit Wasser mischbaren, organischen Lösungsmittel bei Temperaturen zwischen 50 und 240° C, gemeinsam mit der 1,5- bis 20-fachen Gewichtsmenge, bezogen auf das Carotinoid eines eßbaren Öls, sowie einem Emulgator, gegebenenfalls unter erhöhtem Druck, rasch löst, aus der erhaltenen molekulardispersen Lösung durch sofortiges Mischen mit einer wäßrigen Lösung eines Schutzkolloids bei Temperaturen zwischen 0° C und 50° C, die hydrophile Lösungsmittelkomponente in die wäßrige Phase überführt, wobei die hydrophobe Ölphase, die das Carotinoid gelöst enthält, als mikrodisperse Phase entsteht, und die erhaltene Zweiphasen-Mischung in an sich bekannter Weist von dem Lösungsmittel und dem Wasser befreit.

3. Verfahren gemäß Anspruch 2, dadurch gekennzeichnet, daß man das Carotinoid bei Temperaturen von 150 bis 200° C löst.

4. Verfahren gemäß Anspruch 2, dadurch gekennzeichnet, daß man das Carotinoid in weniger als 1 Sekunde löst und die erhaltene Lösung sofort durch Mischen mit der wäßrigen Lösung des Schutzkolloids abkühlt und in die mikrodisperse Zweiphasenmischung überführt.

## Claims

1. A pulverulent, water-dispersable carotenoid formulation which contains from 5 to 50% by weight of an edible oil and in which the carotenoid is dissolved in the edible oil and the oil solution is in the form of small droplets which are dispersed as a pulverulent matrix, obtainable by rapidly dissolving a carotenoid in a volatile, water-miscible, organic solvent at from 50 to 240° C, together with from 1.5 to 20 times the weight, based on the carotenoid, of an edible oil, and an emulsifier, under atmospheric or superatmospheric pressure, then immediately mixing with an aqueous solution of a protective colloid at from 0 to 50° C and thus transferring the hydrophilic solvent component to the aqueous phase, the hydrophobic oil phase which contains the carotenoid in solution being formed as the microdisperse phase, and freeing the resulting two-phase mixture from the solvent and the water in a conventional manner.

2. A process for the preparation of a pulverulent water-dispersable carotenoid formulation which contains from 5 to 50% by weight of an edible oil and in which the carotenoid is dissolved in the edible oil and the oil solution is in the form of small droplets, wherein a carotenoid is rapidly dissolved in a volatile, water-miscible, organic solvent at from 50 to 240° C, together with from 1.5 to 20 times the weight, based on the carotenoid, of an edible oil, and an emulsifier, under atmospheric or superatmospheric pressure, the hydrophilic solvent component is transferred to the aqueous phase from the resulting molecular disperse solution by immediate mixing with an aqueous solution of a protective colloid at from 0 to 50° C, the hydrophobic oil phase which contains the carotenoid in solution being formed as the microdisperse phase, and the resulting two-phase mixture is freed from the solvent and the water in a conventional manner.

3. A process as claimed in claim 2, wherein the carotenoid is dissolved at from 150 to 200° C.

4. A process as claimed in claim 2, wherein the carotenoid is dissolved in less than 1 second, and the resulting solution is immediately cooled by mixing with the aqueous solution of the protective colloid and converted to the microdisperse two-phase mixture.

## Revendications

1. Préparations de carotinoïde pulvérulentes, dispersables dans l'eau, qui contiennent 5 à 50 % en poids d'une huile comestible et dans lesquelles le carotinoïde est dissous dans l'huile comestible et la solution huileuse se trouve sous forme de fines gouttelettes dispersées dans une matrice pulvérulente, pouvant être obtenues par dissolution rapide d'un carotinoïde dans un solvant organique volatil, miscible avec l'eau, à des températures comprises entre 50 et 240 degrés C, éventuellement sous pression, simultanément avec une quantité d'huile comestible 1,5 à 20 fois le poids du carotinoïde, ainsi qu'avec un émulsifiant, mélange immédiatement après avec une solution aqueuse d'un protecteur colloïdal à des températures comprises entre 0 degré C et 50 degrés C et, partant, transfert du composant de solvant hydrophile dans la phase aqueuse, la phase huileuse hydrophobe, qui contient le carotinoïde à l'état dissous se formant alors comme phase microdispersée, et séparation du solvant et de l'eau, de manière en soi connue, d'avec le mélange à deux phases ainsi obtenu.

2. Procédé d'obtention de préparations de carotinoïde pulvérulentes, dispersables dans l'eau, qui contiennent 5 à 50 % en poids d'une huile comestible et dans lesquelles le carotinoïde est dissous dans l'huile comestible et la solution huileuse se trouve sous forme de fines gouttelettes, caractérisé par le fait que l'on dissout rapidement un carotinoïde dans un solvant organique volatil, miscible avec l'eau, à des températures comprises entre 50 et 240 degrés C, éventuellement sous pression, simultanément avec une quantité d'huile comestible 1,5 à 20 fois le poids du carotinoïde, ainsi qu'avec un émulsifiant, on transfère à partir de la solution en dispersion moléculaire ainsi obtenue, par mélange immédiat avec une solution aqueuse d'un protecteur colloïdal, à des températures comprises entre 0 degré C et 50 degrés C, le composant de solvant hydrophile dans la phase aqueuse, la phase huileuse hydrophobe, qui contient le carotinoïde à l'état dissous, se formant alors comme phase microdispersée, et on débarrasse le mélange à deux phases ainsi obtenu, de manière en soi connue, du solvant et de l'eau.

3. Procédé selon la revendication 2, caractérisé par le fait que l'on dissout le carotinoïde à des températures comprises entre 150 et 200 degrés C.

4. Procédé selon la revendication 2, caractérisé par le fait que l'on dissout le carotinoïde en moins de 1 seconde et on refroidit aussitôt la solution ainsi obtenue en la mélangeant avec la solution aqueuse du protecteur colloïdal et on la transfère dans le mélange à deux phases microdispersé.

FIG.1

FIG.2